# EUROPÄISCHE PATENTANMELDUNG

(11) **EP 4 599 775 A1**
(43) Veröffentlichungstag der Anmeldung: **13.08.2025**
(21) Anmeldenummer: 25155710.4
(22) Anmeldetag: 04.02.2025
(51) Int. Cl.: A61B 17/00, A61B 34/37, A61B 34/30

(54) **SCHNITTSTELLE ZUR VERBINDUNG EINER ANTRIEBSEINHEIT MIT EINEM MEDIZINISCHEN INSTRUMENT**

(30) Priorität: 05.02.2024 DE 102024103154
(71) Anmelder: Karl Storz SE & Co. KG, 78532 Tuttlingen (DE)
(72) Erfinder: Klingels, Torben, 84034 Landshut (DE); Hoffmann, Martin, 13187 Berlin (DE)

(57) **Zusammenfassung**

Die Erfindung betrifft eine Schnittstelle (10) zur Verbindung einer Antriebseinheit (12) mit einem medizinischen Instrument (14) umfassend:
- wenigstens ein antriebsseitiges Verbindungselement (16) mit wenigstens einer ersten Kopplungsfläche (18), und
- wenigstens ein instrumentenseitiges Verbindungselement (20) mit wenigstens einer zweiten Kopplungsfläche (22),
wobei die beiden Verbindungselemente (16, 20) über die jeweiligen Kopplungsflächen (18, 22) unter Zwischenschaltung einer durchgängigen medizinischen Barriere (24) miteinander koppelbar sind, und
- wenigstens eine Haltevorrichtung (26), die dazu eingerichtet ist, die Verbindungselemente (16, 20) in einem gekoppelten Zustand zusammenzuhalten.

## Beschreibung

Die vorliegende Erfindung betrifft eine Schnittstelle zur Verbindung einer Antriebseinheit mit einem medizinischen Instrument, insbesondere als Teil eines medizinischen Robotersystems.

Medizinische Robotersysteme, insbesondere in Form von herkömmlichen medizinischen Master-Slave Robotersystemen, die beispielsweise für die mechanische Führung von medizinischen und/oder chirurgischen Instrumenten sowie die Steuerung der Endeffektoren eingesetzt werden, weisen in der Regel eine lösbare Verbindung an einer Schnittstelle zwischen motorischen Antrieben auf der Roboterseite und den chirurgischen Instrumenten auf. Dabei werden die Antriebseinheit und das chirurgische Instrument meist über eine Getriebeanordnung kraftschlüssig miteinander gekoppelt.

Um Anforderungen an eine sterile Behandlungsumgebung erreichen zu können, ist im Bereich der Schnittstelle eine medizinische Barriere umfassend eine Kunststofffolie vorgesehen, welche einen antriebsseitigen Teil der Schnittstelle von einem instrumentenseitigen Teil der Schnittstelle trennt. Zur Übertragung zumindest einer Antriebskraft und/oder zumindest eines Antriebsdrehmoments zwischen den Teilen der Schnittstelle weisen bekannte medizinische Barrieren Einleger und/oder speziell eingebettete Übertragungselemente in der Folie auf, über welchen die Antriebskraft und/oder das Antriebsdrehmoment übertragbar sind. Die Einleger und/oder Übertragungselemente verteuern die medizinische Barriere und diese müssen zudem punktgenau platziert werden, wodurch sich ein Arbeitsaufwand im Vorfeld eines medizinischen und/oder chirurgischen Eingriffs erhöht.

Ausgehend vom Stand der Technik liegt der Erfindung insbesondere aber nicht beschränkt darauf die Aufgabe zugrunde, eine Schnittstelle zur Verbindung einer Antriebseinheit mit einem medizinischen Instrument vorteilhaft weiterzuentwickeln, insbesondere hinsichtlich von Kosten und/oder einer Handhabung.

Diese Aufgabe wird erfindungsgemäß gelöst durch eine Schnittstelle zur Verbindung einer Antriebseinheit mit einem medizinischen Instrument, wie sie hierin beschrieben und in den Ansprüchen definiert ist.

Die Erfindung betrifft eine Schnittstelle zur Verbindung einer Antriebseinheit mit einem medizinischen Instrument umfassend:
- wenigstens ein antriebsseitiges Verbindungselement mit wenigstens einer ersten Kopplungsfläche, und
- wenigstens ein instrumentenseitiges Verbindungselement mit wenigstens einer zweiten Kopplungsfläche,
wobei die beiden Verbindungselemente über die jeweiligen Kopplungsflächen unter Zwischenschaltung einer durchgängigen medizinischen Barriere miteinander koppelbar sind, und
- wenigstens eine Haltevorrichtung, die dazu eingerichtet ist, die Verbindungselemente in einem gekoppelten Zustand zusammenzuhalten.

Durch eine derartige Ausgestaltung kann eine vorteilhaft weiterentwickelte Schnittstelle bereitgestellt werden. Insbesondere kann eine Handhabung vereinfacht und es können Kosten reduziert werden, da eine durchgängige medizinische Barriere zum Einsatz kommt, die insbesondere frei von speziellen Einlegern und/oder speziell eingebetteten Übertragungselementen zur Übertragung einer Antriebskraft und/oder eines Antriebsdrehmoments ist. Hierdurch kann eine aufwändige präzise Platzierung der medizinischen Barriere entfallen. Zudem kann ein Kostenaufwand für jeden medizinischen Einsatz reduziert werden, da eine nur einmalig verwendbare medizinische Barriere möglichst kostengünstig gehalten werden kann. Insbesondere kann eine medizinische Standardbarriere zum Einsatz kommen.

Die Schnittstelle kann Teil eines medizinische Robotersystems sein, welches zusätzlich die Antriebseinheit zur Bereitstellung zumindest einer Antriebskraft und/oder zumindest eines Antriebsdrehmoments und/oder das medizinische Instrument aufweisen kann. Das Robotersystem kann zudem zumindest einen Roboterarm, eine Bedienkonsole, einen Elektronikschrank, insbesondere ein Elektronik-Rack, beispielsweise zur Aufnahme zusätzlicher Geräte wie eines HF-Generators, eine Anzeigeeinheit, eine Patientenliege, eine Aufbewahrungseinheit für verschiedene medizinische Instrumente zur Verwendung mit der Schnittstelle und/oder weitere, dem Fachmann als sinnvoll erscheinende Einheiten aufweisen. Die durchgängige medizinische Barriere kann ebenfalls Teil des medizinischen Robotersystem sein. In einem Betriebszustand kann die Schnittstelle durch den Roboterarm getragen und durch diesen bewegbar sein.

Bei dem medizinischen Instrument kann es sich um ein beliebiges, dem Fachmann als sinnvoll erscheinendes medizinisches und/oder chirurgisches Instrument handeln, welches bevorzugt durch den Roboterarm getragen und durch diesen bewegbar sein kann. Das medizinische Instrument kann mit dem instrumentenseitigen Verbindungselement fest oder lösbar verbunden sein oder Teil des instrumentenseitigen Verbindungselements sein. Das medizinische Instrument kann beispielsweise eine laparoskopische Einheit, ein Endoskop, ein Mikroskop, ein Exoskop, ein Skalpell, einen Bohrer, einen Schaber, eine Klammer, eine Zange, eine Schere, eine Spritze, einen Katheder und/oder weitere, dem Fachmann als sinnvoll erscheinende Einheiten aufweisen, welche mittels der Antriebseinheit über die Schnittstelle antreibbar sein können.

In dem gekoppelten Zustand der Schnittstelle kontaktiert die erste Kopplungsfläche eine erste Seite der medizinischen Barriere und die zweite Kopplungsfläche eine der ersten Seite gegenüberliegende zweite Seite der medizinischen Barriere. Die Kopplungsflächen können dabei bevorzugt zueinander komplementär ausgestaltet sein, insbesondere um eine formschlüssige Aufnahme und/oder ein formschlüssiges Einklemmen der medizinischen Barriere zwischen den Verbindungselementen zu ermöglichen. Die Kopplungsflächen können jeweils eine Gesamtfläche von zumindest 50 mm², insbesondere von zumindest 100 mm² und beispielsweise von zumindest 250 mm² aufweisen.

Die Verbindungselemente können mit ihren Kopplungsflächen zusammengesetzt einen um eine Drehsymmetrieachse zumindest teilweise rotationssymmetrischen, insbesondere zumindest teilweise kreiszylindrischen und/oder wenigstens teilweise kegelstumpfförmigen, Körper ausbilden. Die Verbindungselemente können dabei zumindest teilweise quer zur Drehsymmetrieachse zusammengesetzt sein. In manchen Ausgestaltungen kann das antriebsseitige Verbindungselement größer ausgestaltet sein als das instrumentenseitige Verbindungselement. Das antriebsseitige Verbindungselement kann einen Aufnahmeraum zur Aufnahme des instrumentenseitigen Verbindungselements aufweisen. In dem gekoppelten Zustand können Kanten der Schnittstelle abgerundet sein, um eine Beschädigung der medizinischen Barriere vorteilhaft zu vermeiden.

Die durchgängige medizinische Barriere ist frei von Öffnungen, Einlegern und/oder speziellen eingebetteten Übertragungselementen zur Übertragung einer Antriebskraft und/oder eines Antriebsmoments. Die durchgängige medizinische Barriere kann über Ihre gesamte Flächenerstreckung eine Dicke von höchstens 1 mm, insbesondere von maximal 0,5 mm und beispielsweise von höchstens 0,1 mm aufweisen. Die durchgängige medizinische Barriere ist bevorzugt flexibel ausgebildet. Besonders bevorzugt ist die durchgängige medizinische Barriere als ein entsprechend bearbeitetes und/oder beschichtetes Gewebe und/oder vorzugsweise als eine Folie, insbesondere als eine Kunststofffolie, ausgebildet.

Eine Übertragung der Antriebskraft und/oder des Antriebsdrehmoments durch die durchgängige medizinische Barriere kann mechanisch und/oder magnetisch erfolgen. Dabei kann das antriebsseitige Verbindungselement ein erstes Magnetelement aufweisen, welches mit einem zweiten Magnetelement des instrumentenseitigen Verbindungselement zur Übertragung der Antriebskraft und/oder des Antriebsdrehmoments magnetisch zusammenwirkt. Entsprechende Antriebselemente, insbesondere Magnetelemente, der Schnittstelle können dabei in einem ersten Aufnahmeraum des antriebsseitigen Verbindungselements und/oder einem zweiten Aufnahmeraum des instrumentenseitigen Verbindungselements angeordnet sein.

Die Haltevorrichtung kann dazu eingerichtet sein, die Verbindungselemente in dem gekoppelten Zustand kraft- und/oder formschlüssig zusammenzuhalten. Sie könnte beispielsweise zusammenwirkende Magnetelemente aufweisen, mittels welchen die Verbindungselemente über eine magnetische Kraft zusammengehalten werden. Eines der Magnetelemente könnte dabei ein permanentmagnetisches Element sein, welches mit einem permanentmagnetischen und/oder ferromagnetischen weiteren Element zusammenwirkt. Alternativ könnte eines oder auch beide der Magnetelemente als ein Elektromagnet ausgebildet sein. Zudem wäre denkbar, dass die Verbindungselemente in dem gekoppelten Zustand mittels einer elektrischen Kraft zusammengehalten werden.

Bevorzugt weist die Haltevorrichtung zumindest ein mechanisches Halteelement zu einer mechanischen Verbindung der beiden Verbindungselemente auf.

In manchen Ausführungen kann die Haltevorrichtung eine Klemmeinheit aufweisen, die dazu eingerichtet ist, in dem gekoppelten Zustand zumindest eines der Verbindungselemente zumindest abschnittsweise, insbesondere entlang einer Umfangsrichtung, zu umgreifen. Hierdurch kann konstruktiv einfach eine Verbindung zwischen den Verbindungselementen bereitgestellt werden. Ferner kann eine Beschädigung der medizinischen Barriere vermieden werden. Bevorzugt umgreift die Klemmeinheit in dem gekoppelten Zustand eines der Verbindungselemente, insbesondere das antriebsseitige Verbindungselement, entlang der Umfangsrichtung vollständig. Die Klemmeinheit kann in dem gekoppelten Zustand beide Verbindungselemente zumindest abschnittsweise umgreifen.

Die Klemmeinheit kann in manchen Ausgestaltungen als eine insbesondere steife Klemmschelle ausgebildet sein. Die Klemmeinheit kann zumindest teilweise eine Zylindermantelfläche ausbilden. Alternativ könnte die Klemmeinheit als ein insbesondere flexibler und/oder gummiartiger Halteriemen, vorzugsweise als ein Ratschengurt, ausgebildet sein, welcher an beiden seiner Enden mit dem instrumentenseitigen Verbindungselement verbunden und/oder verbindbar sein könnte, beispielsweise über metallische Ringe an den beiden Enden des Halteriemens, die in entsprechende Haken am instrumentenseitigen Verbindungselement eingehängt werden können. Hierdurch kann eine einfache Konstruktion erreicht werden.

Zudem kann die Klemmeinheit an dem instrumentenseitigen Verbindungselement angeordnet ist, wodurch eine Bedienbarkeit aus einem sterilen Bereich heraus ermöglicht werden kann, insbesondere während eines medizinischen und/oder chirurgischen Eingriffs, insbesondere zu einem Austausch des instrumentenseitigen Verbindungselements gegen ein anderes instrumentenseitiges Verbindungselement. Ferner kann hierdurch ein vorteilhafter Schutz der medizinischen Barriere ermöglicht werden, da sämtliche Elemente der Haltevorrichtung auf einer Instrumentenseite der medizinischen Barriere vorgesehen werden können.

In einigen Ausgestaltungen der Erfindung kann die Klemmeinheit in Umfangsrichtung der Verbindungselemente beweglich mittels einer Führungseinheit des instrumentenseitigen Verbindungselements zwischen einer Freigabeposition und einer Klemmposition geführt sein. Hierdurch kann eine sichere Bedienbarkeit ermöglicht werden. Insbesondere können Fehlbedienungen und/oder ein Verkanten vermieden werden. Die Führungseinheit kann als eine Führungsnut ausgebildet sein, in welche die Klemmeinheit zumindest teilweise eingreift. Die Führungsnut könnte einen T-förmigen Querschnitt aufweisen, in welche ein Führungsteil der Klemmeinheit mit einem passenden Querschnitt formschlüssig eingreift. Bevorzugt erstreckt sich die Führungseinheit, insbesondere die Führungsnut, in Umfangsrichtung über einen Großteil, insbesondere über zumindest 50 %, vorzugsweise über wenigstens 75 % und besonders vorteilhaft über mindestens 90 %, des instrumentenseitigen Verbindungselements.

Vorteilhaft kann die Haltevorrichtung, insbesondere an dem instrumentenseitigen Verbindungselement, eine Arretiereinheit umfassen, die dazu eingerichtet ist, die Klemmeinheit in einer Klemmposition, insbesondere in der zuvor genannten Klemmposition, reversibel zu fixieren. In der Klemmposition kann die Klemmeinheit demzufolge an beiden ihrer Enden an dem instrumentenseitigen Verbindungselement fixiert sein, wobei die Klemmeinheit in einem dazwischen liegenden Bereich an dem antriebsseitigen Verbindungselement anliegen und dieses insbesondere überspannen kann. Hierdurch kann eine vorteilhafte Klemmwirkung erreicht werden. Zudem kann ein sicherer Verschluss der Klemmeinheit bereitgestellt werden.

Die Arretiereinheit kann wenigstens ein Rastmittel, insbesondere eine Rastnase, aufweisen, welche in der Klemmposition mit zumindest einem Gegenrastmittel, insbesondere einer Rastausnehmung, der Klemmeinheit verrastet. In der Freigabeposition kann das Rastmittel, insbesondere die Rastnase, durch die Klemmeinheit ausgelenkt, insbesondere niedergedrückt, sein. Die Arretiereinheit kann in dem gekoppelten Zustand von einer Instrumentenseite der medizinischen Barriere bedienbar, insbesondere lösbar, sein, sodass ein Lösen der Schnittstelle, insbesondere zum Austausch von medizinischen Instrumenten, von einer sterilen Seite her erfolgen kann. Alternativ oder zusätzlich kann die Arretiereinheit zumindest einen Kniehebel oder einen anderem, dem Fachmann als sinnvoll erscheinenden Mechanismus zur Fixierung der Klemmeinheit aufweisen.

Die Klemmeinheit kann vorteilhaft dazu eingerichtet sein, die medizinische Barriere zwischen sich und einer Außenfläche des antriebsseitigen Verbindungselements einzuklemmen. Hierdurch kann eine vorteilhafte Fixierung der Verbindungselemente relativ zu der medizinischen Barriere erreicht werden. In dem gekoppelten Zustand kann die Klemmeinheit abschnittsweise zwischen zwei Lagen der medizinischen Barriere angeordnet sein.

In manchen Ausführungen der Erfindung und insbesondere in bestimmten Stellung en der Klemmeinheit kann eine Innenfläche der Klemmeinheit zumindest abschnittsweise parallel zu einer Außenfläche des instrumentenseitigen Verbindungselements und/oder einer Außenfläche des antriebsseitigen Verbindungselements verlaufen, wodurch eine sichere Führung erreicht werden kann. Zudem kann ein Aufnahmespalt für die medizinische Barriere entlang der Umfangsrichtung in seinen Ausmaßen konstant ausfallen.

Ferner kann das antriebsseitige Verbindungselement und/oder das instrumentenseitige Verbindungselement eine Aufnahmenut aufweisen, in welcher die Klemmeinheit zumindest teilweise und vorzugsweise vollständig aufgenommen ist, wodurch ein vorteilhafter Schutz der Klemmeinheit erreicht werden kann. Zudem kann eine Fixierung der medizinischen Barriere verbessert werden. Die Aufnahmenuten können aneinander anschließend angeordnet sein und sich in Umfangsrichtung vollständig um die Verbindungselemente erstrecken. In dem gekoppelten Zustand kann die medizinische Barriere zumindest teilweise in der Aufnahmenut des antriebsseitigen Verbindungselements angeordnet sein. Kanten der Aufnahmenut des antriebsseitigen Verbindungselements und/oder des instrumentenseitigen Verbindungselements können abgerundet sein, um eine Beschädigung der medizinischen Barriere vorteilhaft zu vermeiden.

In einem Verfahren zum Verbinden der zuvor beschriebenen Schnittstelle werden bevorzugt folgende Schritte durchgeführt:
- zumindest abschnittsweises Bedecken der Kopplungsfläche und/oder der Außenfläche des antriebsseitigen Verbindungselements mit der durchgängigen medizinischen Barriere,
- Koppeln der antriebsseitigen Kopplungsfläche mit der instrumentenseitigen Kopplungsfläche unter Zwischenschaltung der medizinischen Barriere, und
- Betätigen der Haltevorrichtung, insbesondere Schließen der Klemmeinheit.

Hierdurch kann eine Handhabung vereinfacht und es können Kosten reduziert werden, da eine durchgängige medizinische Barriere zum Einsatz kommen kann, die insbesondere frei von speziellen Einlegern und/oder speziell eingebetteten Übertragungselementen zur Übertragung einer Antriebskraft und/oder eines Antriebsdrehmoments ist. Hierdurch kann eine aufwändige präzise Platzierung der medizinischen Barriere entfallen. Zudem kann ein Kostenaufwand für jeden medizinischen Einsatz reduziert werden, da eine nur einmalig verwendbare medizinische Barriere möglichst kostengünstig gehalten werden kann.

Die hierin beschriebenen Vorrichtungen und Systeme sowie das hierin beschriebene Verfahren sollen nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf eine Vorrichtung beschriebenen Merkmale und Eigenschaften, aber auch Verfahrensweisen, sinngemäß auf Verfahren übertragbar und im Sinne der Erfindung einsetzbar und als mitoffenbart gelten. Gleiches gilt auch in umgekehrter Richtung. Das bedeutet, dass auch in Bezug auf Verfahren genannte, bauliche also vorrichtungsgemäße Merkmale im Rahmen der Vorrichtungsansprüche berücksichtigt, beansprucht und ebenfalls zur Offenbarung gezählt werden können.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Es zeigen:
- Fig. 1: ein medizinisches Robotersystem mit einer Schnittstelle zur Verbindung einer Antriebseinheit mit einem medizinischen Instrument,
- Fig. 2: eine perspektivische Darstellung der Schnittstelle mit einem antriebsseitigen Verbindungselement und einem instrumentenseitigen Verbindungselement in einem aneinander angelegten Zustand und mit einer Klemmeinheit in einer Freigabeposition,
- Fig. 3: eine seitliche Darstellung des antriebsseitigen Verbindungselements und des instrumentenseitigen Verbindungselements in einem räumlich voneinander getrennten Zustand,
- Fig. 4: eine perspektivische Darstellung der Schnittstelle bei eingelegter medizinischer Barriere mit der Klemmeinheit in der Freigabeposition,
- Fig. 5: eine perspektivische Darstellung der Schnittstelle bei eingelegter medizinischer Barriere mit der Klemmeinheit in der Klemmposition und
- Fig. 6: ein Ablaufdiagramm eines Verfahrens zum Verbinden der Verbindungselemente unter Zwischenlage der medizinischen Barriere.

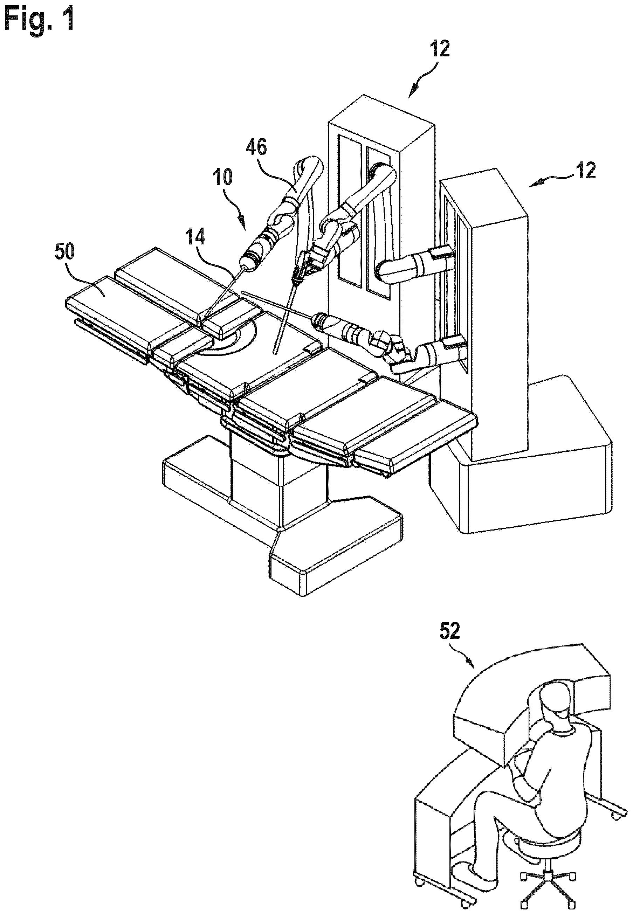

Fig. 1 zeigt ein medizinisches Robotersystem. Das Robotersystem weist eine Patientenliege 50 auf. Das Robotersystem weist einen Roboterarm 46 und eine Antriebseinheit 12 auf, wobei der Roboterarm 46 über die Antriebseinheit 12 steuerbar ist. Eine Ansteuerung der Antriebseinheit 12 kann automatisiert, teilautomatisiert und/oder durch medizinisches Personal mittels einer Bedienkonsole 52 erfolgen.

Das Robotersystem weist ein medizinisches Instrument 14 auf, welches an dem Roboterarm 46 angeordnet und durch diesen bewegbar ist. Das medizinische Instrument 14 selbst weist zumindest ein bewegliches Teil auf, welches gegenüber einem weiteren Teil des medizinischen Instruments 14 beweglich ist. Das bewegliche Teil ist durch die Antriebseinheit 12 relativ zu dem weiteren Teil bewegbar. Vorliegend handelt es sich bei dem medizinischen Instrument 14 um eine Zange, wobei auch beliebige andere, dem Fachmann als sinnvoll erscheinende Instrumente denkbar wären.

Um sterile Arbeitsbedingungen zu schaffen, ist eine durchgängige medizinische Barriere 24 vorgesehen, welche einen Bereich um die Patientenliege 50 von der Antriebseinheit 12 und dem Roboterarm 46 trennt. Eine Antriebskraft und/oder eine Antriebsdrehmoment für das bewegliche Teil des medizinische Instrument 14 wird mittels einer Schnittstelle 10 des Robotersystems übertragen. Die Schnittstelle 10 ist in einem Einsatzzustand im Bereich der medizinischen Barriere 24 angeordnet. Die Schnittstelle 10 ist in dem Einsatzzustand durch den Roboterarm 46 gehalten und geführt.

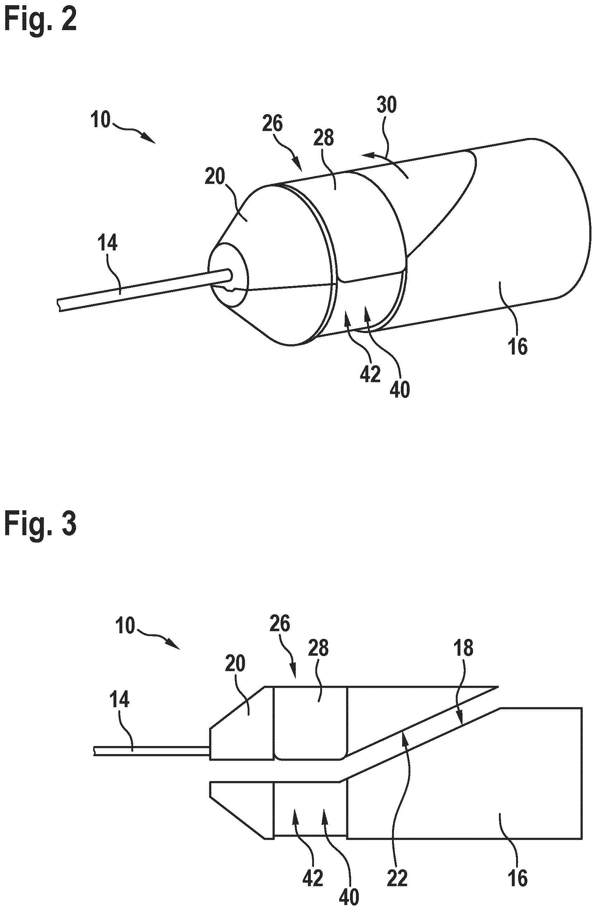

Die Schnittstelle 10 weist ein antriebsseitiges Verbindungselement 16 und ein instrumentenseitiges Verbindungselement 20 auf, welche in Fig. 2 in einer perspektivischen Darstellung in einem aneinander angelegten Zustand gezeigt sind. Fig. 3 zeigt die Verbindungselemente 16, 20 in einem voneinander räumlich getrennten Zustand. Das medizinische Instrument 14 ist fest mit dem instrumentenseitigen Verbindungselement 20 verbunden. Alternativ wäre auch eine lösbare Verbindung des medizinischen Instruments 14 mit dem instrumentenseitigen Verbindungselement 20 denkbar.

Das antriebsseitige Verbindungselement 16 weist eine erste Kopplungsfläche 18 auf. Das instrumentenseitige Verbindungselement 20 umfasst eine zweite Kopplungsfläche 22. Die Kopplungsflächen 18, 22 sind zueinander komplementär ausgebildet und erlauben ein formschlüssiges Zusammenfügen der Verbindungselemente 16, 20, wie in Fig. 2 dargestellt.

Die Verbindungselemente 16, 20 bilden zusammengesetzt einen zumindest teilweise kreiszylindrischen und zumindest teilweise kegelstumpfförmigen Körper. Das antriebsseitige Verbindungselement 16 ist größer ausgestaltet als das instrumentenseitige Verbindungselement 20 und weist einen Aufnahmeraum zur Aufnahme des instrumentenseitigen Verbindungselements 20 auf (vgl. Fig. 3).

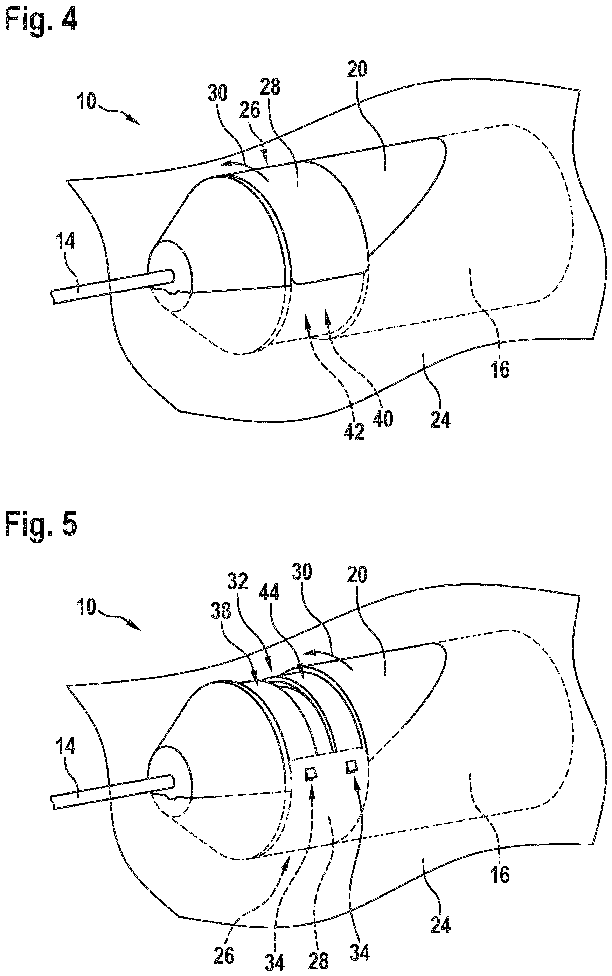

Die Kopplungsflächen 18, 22 sind dazu eingerichtet, die medizinische Barriere 24 einzuklemmen (vgl. Fig. 4 und 5). Die Verbindungselemente 16, 20 sind über ihre jeweiligen Kopplungsflächen 18, 22 unter Zwischenschaltung der medizinischen Barriere 24 miteinander koppelbar. Bei der medizinischen Barriere 24 handelt es sich um eine durchgängige Kunststofffolie, welche insbesondere frei von Öffnungen ist. In dem Einsatzzustand sind die beiden Verbindungselemente 16, 20 mittels der medizinischen Barriere 24 voneinander getrennt. Dabei befindet sich das instrumentenseitige Verbindungselement 20 zusammen mit dem medizinischen Instrument 14 in dem Einsatzzustand auf einer sterilen Seite der medizinischen Barriere 24.

Eine Übertragung einer Antriebskraft und/oder eines Antriebsdrehmoments durch die durchgängige medizinische Barriere 24 erfolgt beispielsweise magnetisch (nicht dargestellt). In diesem Fall weist das antriebsseitige Verbindungselement 16 in seinem Innern ein erstes Magnetelement auf, welches mit einem zweiten Magnetelement des instrumentenseitigen Verbindungselement 20 zur Übertragung der Antriebskraft und/oder des Antriebsdrehmoments magnetisch zusammenwirkt. Das erste Magnetelement ist dabei durch die Antriebseinheit 12 antreibbar.

Die Schnittstelle 10 weist eine Haltevorrichtung 26 auf, die dazu eingerichtet ist, die Verbindungselemente 16, 20 in einem gekoppelten Zustand und insbesondere in dem Einsatzzustand zusammenzuhalten.

Die Haltevorrichtung 26 weist eine Klemmeinheit 28 auf, die dazu eingerichtet ist, in dem gekoppelten Zustand und insbesondere in dem Einsatzzustand zumindest eines der Verbindungselemente 16, 20 zumindest abschnittsweise zu umgreifen. Die Klemmeinheit 28 ist dabei dazu eingerichtet, in dem gekoppelten Zustand und insbesondere in dem Einsatzzustand das antriebsseitige Verbindungselement 16 in Umfangsrichtung 30 der Verbindungselemente 16, 20 vollständig zu umgreifen. Die Klemmeinheit 28 ist als eine Klemmschelle ausgebildet und bildet zumindest teilweise eine Zylindermantelfläche aus.

Die Klemmeinheit 28 ist an dem instrumentenseitigen Verbindungselement 20 angeordnet. Die Klemmeinheit 28 ist in Umfangsrichtung 30 beweglich mittels einer Führungseinheit 32 (vgl. Fig. 5) des instrumentenseitigen Verbindungselements 20 zwischen einer Freigabeposition gemäß Fig. 4 und einer Klemmposition gemäß Fig. 5 geführt. Bei Überführung der Klemmeinheit 28 von der Freigabeposition in die Klemmposition ist die Klemmeinheit 28 entlang der Umfangsrichtung 30, und zwar in der in Fig. 2 gezeigten Pfeilrichtung, um die Verbindungselemente 16, 20 herumzubewegen. Eine Innenfläche der Klemmeinheit 28 verläuft dabei zumindest abschnittsweise parallel zu einer Außenfläche 38 des instrumentenseitigen Verbindungselements 20 und einer Außenfläche 40 des antriebsseitigen Verbindungselements 16.

Die Führungseinheit 32 ist als eine Führungsnut ausgebildet, in welche die Klemmeinheit 28 zumindest teilweise eingreift. Die Führungsnut weist einen T-förmigen Querschnitt auf, in welchen ein Führungsteil der Klemmeinheit 28 mit einem passenden Querschnitt formschlüssig eingreift (nicht dargestellt). Die Führungsnut erstreckt sich in Umfangsrichtung fast das gesamte instrumentenseitige Verbindungselement 20.

Die Haltevorrichtung 26 umfasst eine Arretiereinheit 34, die dazu eingerichtet ist, die Klemmeinheit 28 in der Klemmposition reversibel zu fixieren. In der Klemmposition ist die Klemmeinheit 28 an ihren beiden Enden an dem instrumentenseitigen Verbindungselement 20 fixiert, wobei die Klemmeinheit 18 in einem dazwischen liegenden Bereich an dem antriebsseitigen Verbindungselement 16 anliegt und dieses überspannt. Die Arretiereinheit 34 weist zwei Rastnasen auf, welche in der Klemmposition mit zwei Rastausnehmungen 48 der Klemmeinheit 28 verrastet sind (vgl. Fig. 5). In der Freigabeposition sind die Rastnasen durch die Klemmeinheit 28 niedergedrückt (nicht dargestellt).

Das antriebsseitige Verbindungselement 16 weist eine Aufnahmenut 42 auf (vgl. Fig. 2, 3 und 4). Das instrumentenseitige Verbindungselement 20 umfasst eine Aufnahmenut 44 (vgl. Fig. 5). Die Aufnahmenuten 42, 44 sind aneinander anschließend angeordnet und erstrecken sich in Umfangsrichtung 30 vollständig um die Verbindungselemente 16, 20. Die Klemmeinheit 28 ist in die Aufnahmenuten 42, 44 bündig aufgenommen und entlang dieser bewegbar. Die Führungseinheit 32 ist in der Aufnahmenut 44 angeordnet.

Die Klemmeinheit 28 ist dazu eingerichtet, die medizinische Barriere 24, wie in Fig. 5 dargestellt, zwischen sich und der Außenfläche 40 des antriebsseitigen Verbindungselements 16 einzuklemmen.

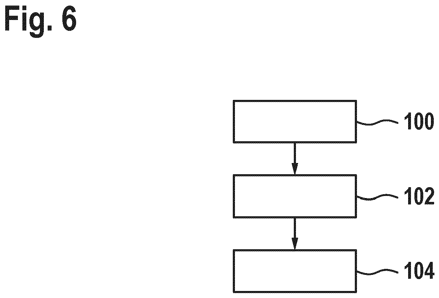

Fig. 6 zeigt ein Ablaufdiagramm eines Verfahrens zum Verbinden der Verbindungselemente 16, 20 unter Zwischenlage der medizinischen Barriere 24. In einem Schritt 100 wird die erste Kopplungsfläche 18 und die Außenfläche 40 des antriebsseitigen Verbindungselements 16 zumindest abschnittsweise mit der medizinischen Barriere 24 bedeckt. In einem Schritt 102 wird das instrumentenseitige Verbindungselement 20 über seine zweite Kopplungsfläche 22 unter Zwischenlage der medizinischen Barriere 24 mit der ersten Kopplungsfläche 18 verbunden. In einem Schritt 104 wird schließlich die Klemmeinheit 28 betätigt.

### Bezugszeichenliste

- 10: Schnittstelle
- 12: Antriebseinheit
- 14: medizinisches Instrument
- 16: antriebsseitiges Verbindungselement
- 18: erste Kopplungsfläche
- 20: instrumentenseitiges Verbindungselement
- 22: zweite Kopplungsfläche
- 24: medizinische Barriere
- 26: Haltevorrichtung
- 28: Klemmeinheit
- 30: Umfangsrichtung
- 32: Führungseinheit
- 34: Arretiereinheit
- 38: Außenfläche
- 40: Außenfläche
- 42: Aufnahmenut
- 44: Aufnahmenut
- 46: Roboterarm
- 48: Rastausnehmung
- 50: Patientenliege
- 52: Bedienkonsole
- 100: Schritt
- 102: Schritt
- 104: Schritt

## Patentansprüche

1. Schnittstelle (10) zur Verbindung einer Antriebseinheit (12) mit einem medizinischen Instrument (14) umfassend:
- wenigstens ein antriebsseitiges Verbindungselement (16) mit wenigstens einer ersten Kopplungsfläche (18), und
- wenigstens ein instrumentenseitiges Verbindungselement (20) mit wenigstens einer zweiten Kopplungsfläche (22),
wobei die beiden Verbindungselemente (16, 20) über die jeweiligen Kopplungsflächen (18, 22) unter Zwischenschaltung einer durchgängigen medizinischen Barriere (24) miteinander koppelbar sind, und
- wenigstens eine Haltevorrichtung (26), die dazu eingerichtet ist, die Verbindungselemente (16, 20) in einem gekoppelten Zustand zusammenzuhalten.

2. Schnittstelle (10) nach Anspruch 1,
wobei die Haltevorrichtung (26) eine Klemmeinheit (28) aufweist, die dazu eingerichtet ist, in dem gekoppelten Zustand zumindest eines der Verbindungselemente (16, 20) zumindest abschnittsweise zu umgreifen.

3. Schnittstelle (10) nach Anspruch 2,
wobei die Klemmeinheit (28) an dem instrumentenseitigen Verbindungselement (20) angeordnet ist.

4. Schnittstelle (10) nach Anspruch 2 oder 3,
wobei die Klemmeinheit (28) in Umfangsrichtung (30) der Verbindungselemente (16, 20) beweglich mittels einer Führungseinheit (32) des instrumentenseitigen Verbindungselements (20) zwischen einer Freigabeposition und einer Klemmposition geführt ist.

5. Schnittstelle (10) nach einem der Ansprüche 2 bis 4,
wobei die Haltevorrichtung (26) eine Arretiereinheit (34) umfasst, die dazu eingerichtet ist, die Klemmeinheit (28) in einer Klemmposition reversibel zu fixieren.

6. Schnittstelle (10) nach einem der Ansprüche 2 bis 5,
wobei eine Innenfläche der Klemmeinheit (28) zumindest abschnittsweise parallel zu einer Außenfläche (38) des instrumentenseitigen Verbindungselements (20) und/oder einer Außenfläche (40) des antriebsseitigen Verbindungselements (16) verläuft.

7. Schnittstelle (10) nach einem der Ansprüche 2 bis 6,
wobei das antriebsseitige Verbindungselement (16) und/oder das instrumentenseitige Verbindungselement (20) eine Aufnahmenut (42, 44) aufweist, in welcher die Klemmeinheit (28) zumindest teilweise aufgenommen ist.

8. Schnittstelle (10) nach einem der Ansprüche 2 bis 7,
wobei die Klemmeinheit (28) dazu eingerichtet ist, die medizinische Barriere (24) zwischen sich und einer Außenfläche (40) des antriebsseitigen Verbindungselements (16) einzuklemmen.

9. Medizinisches Robotersystem umfassend:
- eine Schnittstelle (10) nach einem der vorhergehenden Ansprüche.

10. Medizinisches Robotersystem nach Anspruch 9, weiter umfassend:
- die Antriebseinheit (12), welche mit dem antriebsseitigen Verbindungselement (16) verbunden ist.

11. Medizinisches Robotersystem nach Anspruch 9 oder 10, weiter umfassend:
- das medizinische Instrument (14), welches mit dem instrumentenseitigen Verbindungselement (20) verbunden ist.

12. Medizinisches Robotersystem nach einem der Ansprüche 9 bis 11, weiter umfassend:
- die medizinische Barriere (24), welche zwischen dem antriebsseitigen Verbindungselement (16) und dem instrumentenseitigen Verbindungselement (20) eingeklemmt ist.
